# EUROPEAN PATENT APPLICATION

(11) **EP 3 501 434 A1**
(43) Date of publication of application: **26.06.2019**
(21) Application number: 18213838.8
(22) Date of filing: 19.12.2018
(51) Int. Cl.: A61B 17/34

(54) **SYSTEM FOR ASPIRATION OF BILE**

(30) Priority: 20.12.2017 US 201762608240 P; 03.12.2018 US 201816207646
(71) Applicant: Covidien LP, Mansfield, MA 02048 (US)
(72) Inventor: LUBINSKI, Alexander A., San Francisco, CA 94110 (US); MAGUIRE, Mark A., Hillsborough, CA 94010 (US); CAMISA, William J., Los Altos, CA 94024 (US); MORRIS, Kevin M., San Francisco, CA 94107 (US)
(74) Representative: Maschio, Antonio

(57) **Abstract**

Methods, apparatuses and systems are described for providing access to a body lumen. The access system may include an access catheter with a lumen extending from a proximal end to a distal end of the access catheter and a stylet configured to fit within the lumen of the access catheter. The stylet may include a lumen extending from a proximal end to a distal end of the stylet and a piercing tip at the distal end of the stylet configured to protrude from the distal end of the access catheter. The access system may be advanced adjacent to the body lumen and the piercing tip of the stylet may pierce the wall of the body lumen by advancing the stylet and the access catheter together.

## Description

Diseases and disorders of the gallbladder, pancreas, and bile ducts (i.e., pancreaticobiliary system) are associated with significant morbidity, mortality, and impaired quality of life. Obstructions, tumors, injuries, leakages, inflammation, infection, and lesions can occur in these structures, which can eventually lead to conditions such as biliary colic, cholecystitis, choledocholithiasis, cholelithiasis, pancreatitis, pancreatic duct stone formations, and chronic abdominal pain. Diseases of the pancreaticobiliary system may also be associated with nutritional disorders, such as malnutrition, obesity, and high cholesterol.

To treat a blockage within the bile duct, a clinician may perform a biliary drainage procedure to restore adequate drainage through the affected duct. A biliary drainage procedure may include endoscopically accessing the bile duct with a catheter that is advanced over a sharpened stylet. Once the bile duct is punctured, the clinician may confirm the location of the catheter by withdrawing the stylet from the catheter and aspirating bile through the catheter.

If the clinician determines that the catheter has been incorrectly placed (e.g., not within the bile duct), the clinician may then have to reposition the catheter, reinsert the stylet through the catheter, re-puncture the bile duct with the stylet, and repeat the aspiration procedure. This technique is labor intensive and may lead to increased discomfort and serious medical complications for the patient.

### SUMMARY

The described features generally relate to improved methods, systems, and devices for accessing a body lumen. A system may include an access catheter and a stylet configured to fit within the access catheter. The stylet may be hollow and include a piercing tip that protrudes from the end of the access catheter. The stylet and access catheter may be advanced together to pierce a wall of the body lumen. The piercing tip may be offset from a circumferential outer surface of the stylet. The hollow stylet may facilitate flushing contrast into and aspirating fluid from a body lumen, thereby reducing or eliminating the procedure of withdrawing the stylet from the access catheter each time a flushing or aspiration procedure is performed.

Methods and apparatuses are described for accessing a body lumen. A method for accessing a body lumen is described. The method may include advancing an access system adjacent to the body lumen, where the access system comprises an access catheter comprising a lumen extending from a proximal end to a distal end of the access catheter and a plurality of apertures disposed along a distal portion of the access catheter. The access system may also comprise a stylet configured to fit within the lumen of the access catheter, the stylet comprising a lumen extending from a proximal end to a distal end of the stylet and a piercing tip at the distal end of the stylet configured to protrude from the distal end of the access catheter. The method may also include piercing a wall of the body lumen with the piercing tip of the stylet by advancing the stylet and the access catheter together.

In some embodiments, the piercing tip is offset from a circumferential outer surface of the stylet. In some embodiments, the method may include aspirating a fluid from the body lumen through the lumen of the stylet. The method may also include flushing a contrast fluid through the lumen of the stylet. Additionally, the stylet may further comprise a plurality of apertures disposed along a distal portion of the stylet, wherein the contrast fluid is flushed through the plurality of apertures of the stylet.

In some embodiments, the access system further comprises a delivery handle assembly, an inner hub housing coupled to a proximal portion of the delivery handle assembly, a sheath coupled to a distal portion of the delivery handle assembly and having a lumen in fluid communication with an inner lumen of the delivery handle assembly, and an access catheter subassembly removably disposed within the inner lumen of the delivery handle assembly and the lumen of the sheath. In some embodiments, the access catheter subassembly may comprise a catheter hub configured for insertion into the inner hub housing, wherein the catheter hub is rotatable with respect to the inner hub housing and the access catheter coupled with the catheter hub.

In some embodiments, the method may include rotating the catheter hub with respect to the inner hub housing. In some cases, the distal portion of the access catheter may comprise a pre-defined arcuate shape. The method may further comprise straightening the distal portion of the access catheter by advancing the stylet through the distal portion of the access catheter.

A system for providing access to a body lumen may include an access catheter comprising a lumen extending from a proximal end to a distal end of the access catheter and a plurality of apertures disposed along a distal portion of the access catheter. The access catheter may also include a stylet configured to fit within the lumen of the access catheter, the stylet comprising a lumen extending from a proximal end to a distal end of the stylet and a piercing tip at the distal end of the stylet.

In some embodiments, the piercing tip is offset from a circumferential outer surface of the stylet. In some embodiments, the stylet may include a first bevel forming a first angle with respect to a longitudinal axis of the stylet, a second bevel forming a second angle with respect to the longitudinal axis of the stylet and abutting the first bevel, wherein the first angle is less than the second angle, and a back-cut bevel forming a back-cut angle with respect to a circumferential outer surface of the stylet, wherein the second bevel and the back-cut bevel abut at the piercing tip.

In some embodiments, the stylet may include a single bevel abutting a non-circumferential outer surface of the stylet at the piercing tip, wherein a distal portion of the stylet is curved such that a center line of the stylet intersects the piercing tip. In some examples, the stylet may include three bevels forming three equal angles with respect to a longitudinal axis of the stylet and each abutting with each other at the piercing tip. In some cases, a center line of the stylet intersects the piercing tip. The stylet may also include a plurality of apertures disposed along a distal portion of the stylet, wherein the plurality of apertures extend from a circumferential outer surface of the stylet to an inner surface of the lumen of the stylet.

In some embodiments, the system for providing access to a body lumen may include a delivery handle assembly, an inner hub housing coupled to a proximal portion of the delivery handle assembly, a sheath coupled to a distal portion of the delivery handle assembly and having a lumen in fluid communication with an inner lumen of the delivery handle assembly, and an access catheter subassembly removably disposed within the inner lumen of the delivery handle assembly and the lumen of the sheath. The access catheter subassembly may comprise a catheter hub configured for insertion into the inner hub housing, wherein the catheter hub is rotatable with respect to the inner hub housing and the access catheter coupled with the catheter hub.

In some embodiments, the system for providing access to a body lumen may include a stylet hub coupled with the proximal end of the stylet and configured to removably couple with the catheter hub, wherein the stylet hub comprises a syringe port in fluid communication with the lumen of the stylet. In some examples, a cross-section of the distal end of the stylet and a cross-section of the proximal end of the stylet are circular. In some cases, the distal portion of the access catheter comprises a pre-defined arcuate shape, and wherein the distal portion of the access catheter is configured to straighten from the pre-defined arcuate shape as the stylet is advanced through the distal portion of the access catheter. In some embodiments, a circumferential outer surface of the stylet comprises a lubricous coating.

Certain examples of the present disclosure may include some, all, or none of the above advantages or features. One or more other technical advantages or features may be readily apparent to those skilled in the art from the figures, descriptions, and claims included herein. Moreover, while specific advantages or features have been enumerated above, various embodiments may include all, some, or none of the enumerated advantages or features.

Further scope of the applicability of the described methods and systems will become apparent from the following detailed description, claims, and drawings. The detailed description and specific examples are given by way of illustration only, since various changes and modifications within the spirit and scope of the description will become apparent to those skilled in the art.

### BRIEF DESCRIPTION OF THE DRAWINGS

In the appended figures, similar components or features may have the same reference label. Further, various components of the same type may be distinguished by following the reference label by a dash and a second label that distinguishes among the similar components. If only the first reference label is used in the specification, the description is applicable to any one of the similar components having the same first reference label irrespective of the second reference label.
FIG. 1 illustrates an exploded view of a system for providing access to a body lumen in accordance with aspects of the present disclosure.
FIG. 2 illustrates a perspective view of an access system with a stylet in accordance with aspects of the present disclosure.
FIG. 3A illustrates a perspective view of a stylet with a beveled tip in accordance with aspects of the present disclosure.
FIG. 3B illustrates a side view of the stylet of FIG. 3A in accordance with aspects of the present disclosure.
FIG. 3C illustrates a top view of the stylet of FIG. 3A in accordance with aspects of the present disclosure.
FIG. 4A illustrates a perspective view of a stylet with a bent tip in accordance with aspects of the present disclosure.
FIG. 4B illustrates a side view of the stylet of FIG. 4A in accordance with aspects of the present disclosure.
FIG. 4C illustrates a top view of the stylet of FIG. 4A in accordance with aspects of the present disclosure.
FIG. 5A illustrates a perspective view of a stylet with a welded tip in accordance with aspects of the present disclosure.
FIG. 5B illustrates a side view of the stylet of FIG. 5A in accordance with aspects of the present disclosure.
FIG. 5C illustrates a top view of the stylet of FIG. 5A in accordance with aspects of the present disclosure.
FIG. 6A illustrates a perspective view of an access system in a linear configuration in accordance with aspects of the present disclosure.
FIG. 6B illustrates a perspective view of the access system in a nonlinear configuration in accordance with aspects of the present disclosure.
FIG. 7 illustrates a system for accessing a body lumen of the pancreaticobiliary system in accordance with aspects of the present disclosure.
FIG. 8 illustrates a system for accessing a body lumen of the pancreaticobiliary system in accordance with aspects of the present disclosure.
FIGs. 9-10 illustrate flow diagrams of methods of accessing a body lumen in accordance with aspects of the present disclosure.

### DETAILED DESCRIPTION

The present disclosure is generally directed to apparatuses, systems, and methods for accessing a body lumen and aspirating fluid from the body lumen for subsequent treatment. In accordance with various examples, a system for accessing a body lumen may include an access catheter and a stylet, where the stylet is configured to fit within a lumen of the access catheter and to work in concert with the access catheter to pierce the luminal wall. In some examples, the system also includes a delivery handle member with one or more features configured to manipulate the axial and rotational movement of the access catheter and stylet, with respect to each other and the accessed body lumen.

The stylet may be configured to pierce the luminal wall for access into the body lumen. For example, the stylet may include a piercing tip that protrudes from the distal end of the access catheter. The stylet and the access catheter may advance together to pierce the luminal wall of the body lumen. In some examples, the piercing tip is offset from a circumferential surface of the stylet, which may reduce catching of the stylet on the inner wall of the access catheter (or an external sheath) as the stylet is advanced through the access catheter or access system. The circumferential offset may be the result of one or more bevel cuts of the distal tip (e.g., a back cut). In some examples, the circumferential offset is achieved by bending the distal tip toward a center line of the stylet. In yet other examples, the distal tip may be centered on the center line of the stylet.

As described with reference to various examples, the stylet may include one or more features designed to flush contrast or aspirate fluid from a body lumen without the removal of the stylet from the access catheter. For example, all or a portion of the stylet may be hollow (e.g., may include a lumen extending from a proximal end to a distal end of the stylet). In some examples, the stylet may include apertures disposed along a portion of the wall of the stylet to further facilitate the aspiration and flushing of fluid through the stylet. The features of the stylet that facilitate aspirating or flushing fluid through the stylet, instead of removing a solid stylet from a catheter to flush or aspirate, may reduce the complexity and risk associated with performing certain luminal access procedures, such as those of the pancreaticobiliary system. For example, in the case of a biliary access procedure, the piercing tip of the stylet may puncture the bile duct, the clinician may aspirate fluid from the bile duct through the stylet and flush contrast through the stylet into the bile duct to confirm proper access location in the bile duct. That is, the location may be confirmed without the removal of the stylet from the lumen of the access catheter. This technique may eliminate the need to reinsert the stylet if the access catheter is positioned adjacent to the wall of the target body lumen. In some cases, penetrating the tissue and confirming the location without removal of the stylet may save the clinician time in performing the procedure and may prevent increased discomfort to the patient.

In various examples, the distal section of the access catheter includes a plurality of apertures to increase the flexibility of the distal section. For example, the shape, size, and pattern of the apertures may be tailored to achieve certain access catheter characteristics such as the shape of the distal section in the nonlinear configuration, a certain stiffness of the distal section, a particular flexibility profile, or resistance to fracture. The apertures may be sized and located along the access catheter to facilitate the advancement of the stylet or guide wire without catching on the apertures. For example, the distal tip of the stylet may be offset from a circumferential surface of the stylet to reduce catching on the apertures of the access catheter as the stylet is advanced through the access catheter. Additionally, the stylet may be inserted into the lumen of the access catheter to prevent leakage of fluid and/or contrast through the apertures when the clinician aspirates fluid through the stylet and flushes contrast through the stylet. In some examples, the same or different shaped apertures may be included on a section of the access catheter proximal to the pre-curved distal section in order to provide flexibility along this section to facilitate advancement of the access catheter and stylet deeper into the body lumen.

Examples of the present disclosure are now described in detail with reference to the drawings. As used herein, the term "clinician" refers to a doctor, surgeon, nurse, or any other care provider and may include support personnel. The term "proximal" will refer to the portion of the device or component thereof that is closer to the clinician and the term 'distal" will refer to the portion of the device or component thereof that is farther from the clinician.

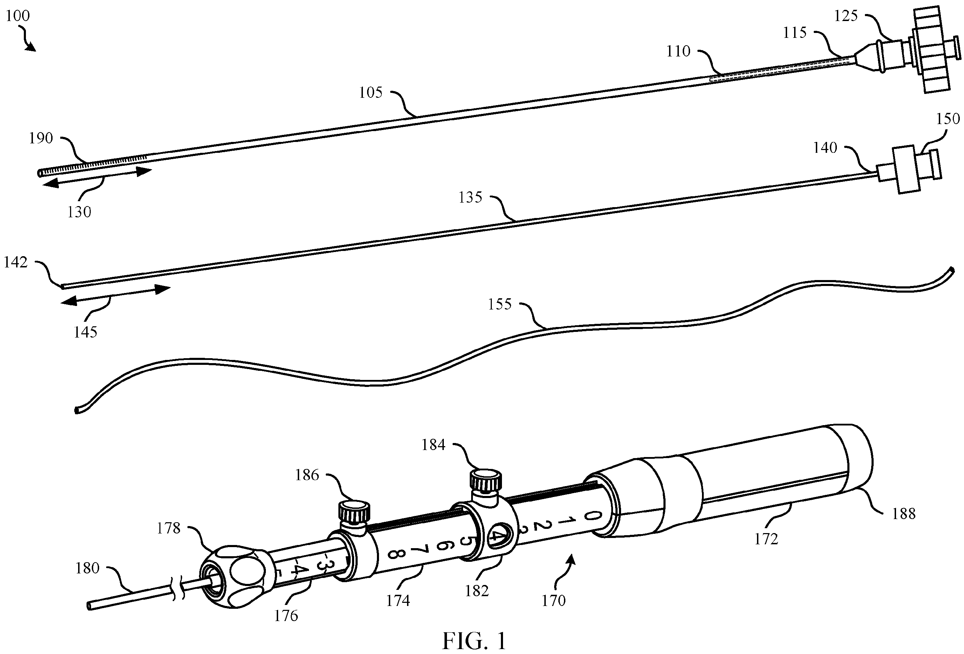

**FIG. 1** illustrates an exploded view of a system 100 for providing access to a body lumen in accordance with aspects of the present disclosure. The system 100 generally includes an access catheter 105, a stylet 135, a guidewire 155, and a delivery handle assembly 170. The system 100 can be provided as individual components, selectively combined components, or all together as a kit of components. The access catheter 105 may be inserted into the delivery handle assembly 170 (through the proximal end 188) until the access catheter hub 125 abuts against the proximal end 188. Once assembled, the access catheter 105 extends through the delivery handle assembly 170 and through the elongate sheath 180 to the target body lumen.

The stylet 135 is generally an elongate, tubular member with proximal end 140, and distal end 142, and distal portion 145, and is dimensioned to fit within and slidably advance through the lumen 110 of the access catheter 105. The stylet 135 may include a sharpened tip (e.g., a piercing tip) at the distal end 142. The stylet 135 may also include a stylet hub 150 coupled with the proximal end 140 of the stylet 135 to facilitate longitudinal manipulation of the stylet 135 with respect to the access catheter 105. In some cases, the stylet hub 150 may be uncoupled from the proximal end 140 of the stylet 135 to facilitate removal of the stylet 135 from the access catheter 105.

The guidewire 155 is generally a flexible elongate member configured to slidably advance through the lumen 110 of the access catheter 105. The guidewire 155 may be uniform in size and stiffness along its entire length, or alternatively, may include sections of differing stiffness.

During a luminal access procedure, the access catheter 105 may access the target lumen by piercing a wall of the lumen, for example. In some examples, the stylet 135 may be used in conjunction with the access catheter 105 to facilitate piercing the luminal wall. For example, the stylet 135 may fit within the access catheter 105 and the piercing tip of the stylet 135 may protrude from the access catheter 105 to pierce tissue. In some cases, the stylet 135 and access catheter 105 may be advanced together to pierce a wall of the body lumen with the piercing tip of the stylet 135. The sharpened distal tip of the stylet 135 may include a plurality of bevels to form a piercing tip offset from a circumferential outer surface of the stylet 135. As such, the body of the stylet 135 may advance through the lumen of the access catheter 105 without catching on the inner surface of the access catheter 105 or an external sheath. In addition, all or a portion of the stylet 135 may be hollow so that fluid may be flushed and aspirated through the stylet 135. A distal portion 145 of the stylet 135 may include apertures disposed along the wall of the stylet 135 to further facilitate in the flushing and aspiration of fluid.

Once the access catheter 105 has accessed the lumen, the clinician may aspirate fluid from the lumen (e.g., bile duct) through the stylet 135 and flush contrast through the stylet 135 into the lumen. After the proper access location in the bile duct is confirmed, the clinician may remove stylet 135 from the access catheter 105. The guidewire 155 may be advanced through the access catheter 105 and into the lumen. After correct placement of the guidewire 155 inside the body lumen, the access catheter 105 may be removed. The guidewire 155 may remain inside the body lumen to allow correct placement of other devices (e.g., a stent).

In some examples, a distal section 130 of the access catheter 105 is configured to curl or curve into a pre-defined arcuate shape (e.g., through heat setting techniques). For example, a distal section 130 of the access catheter 105 may include one or more apertures 190 that are sized, arranged, or otherwise configured to facilitate flexing or bending of the distal section 130 to the nonlinear shape. The stylet 135 and access catheter 105 may be configured such that as the stylet 135 is advanced through the curved distal section 130 of the access catheter 105, the distal section 130 of the access catheter 105 straightens out. This may be achieved by tailoring the relative stiffness of the distal section 130 of the access catheter 105 with respect to the stylet 135.

For example, a distal section 130 of the access catheter 105 may be configured to passively transition (i.e., naturally move in the absence of constraining forces) from a linear shape into a nonlinear shape within a body lumen, and vice versa. For example, in accordance with various examples, the distal section 130 may be constrained in a linear configuration by an internal member (e.g., a stylet 135) or an external member (e.g., a elongate sheath 180), and as the member is withdrawn (e.g., translated axially with respect to the access catheter 105 in the proximal direction), the distal section 130 may then passively transition into a nonlinear configuration.

In accordance with various examples, one or more apertures 190 are disposed along the distal section 130 and are sized, arranged, or otherwise configured to facilitate flexing or bending of the distal section 130 to the nonlinear shape. In some cases, a variety of shapes and patterns of the apertures 190 may be used to impart certain flexibility characteristics to the access catheter 105. In some examples, the apertures 190 may extend proximal to distal section 130, thereby providing flexibility to portions of the access catheter 105 that are not configured to passively transition into a nonlinear shape.

The delivery handle assembly 170 is generally configured to facilitate manipulation of the access catheter 105, the stylet 135, and the guidewire 155 with respect to each other, the accessed body lumen, or an attached endoscope. The delivery handle assembly 170 may include a proximal handle member 172 with a proximal end 188, a middle handle member 174, and a distal handle member 176. The proximal, middle, and distal handle members 172, 174, 176 each include an inner lumen and are coupled together to form a continuous lumen extending throughout the length of the delivery handle assembly 170. The proximal handle member 172 is slidably disposed over at least a portion of the middle handle member 174, and, similarly, the middle handle member 174 is slidably disposed over at least a portion of distal handle member 176. The distal handle member 176 may also include a threaded connector element 178 configured to securely attach to a working channel of an endoscope (not shown).

One or more of the proximal, middle, and distal handle members 172, 174, and 176 of the delivery handle assembly 170 may be configured to manipulate one or more components of a bile aspiration system. For example, a proximal hub may be configured to advance and retract the stylet 135 with respect to the elongate sheath 180. A middle hub may be configured to advance and retract the sheath itself. As such, the middle hub may facilitate advancement of the elongate sheath 180 and manipulation of the elongate sheath 180 with respect to a body lumen.

The delivery handle assembly 170 may also include an elongate sheath 180 extending from the distal end of the distal handle member 176. The elongate sheath 180 is generally made from a flexible polymeric material and provides a continuous conduit through which the access catheter 105 or other elements (e.g., a stylet 135) may travel between the delivery handle assembly 170 and the target tissue within the body (e.g., the bile duct). Accordingly, the length and diameter of the elongate sheath 180 may depend upon the particular application.

The delivery handle assembly 170 may also include one or more adjustment features that limit the sliding movement of the proximal, middle, and distal handle members 172, 174, 176 relative to each other. For instance, the delivery handle assembly 170 may include a locking ring 182 with a threaded thumbscrew 184 disposed around the middle handle member 174. The locking ring 182 may be slid along the middle handle member 174 and tightened in a desired position with the threaded thumbscrew 184. When tightened, the locking ring 182 limits the movement of the proximal handle member 172 in the distal direction relative to the middle handle member 174, thereby allowing the clinician to establish a set penetration depth of the access catheter 105 or stylet 135 beyond the distal end of the elongate sheath 180. Similarly, a thumbscrew 186 is configured to lock the position of the distal handle member 176 with respect to the middle handle member 174, thereby allowing the clinician to set an extension depth of the elongate sheath 180 beyond the distal end of an attached endoscope.

The system 100 may be used to access and provide treatment to one or more body lumens within the gastrointestinal system or pancreaticobiliary system, for example. It may be appreciated that the system 100 may also be used to provide access or treatment to other organs or luminal systems within the body such as the arterial system, the bronchial system, the urinary system, or any other luminal system were maneuverability and accuracy is desirable.

In some examples described herein, the delivery handle assembly 170 is coupled with an endoscope and the access catheter 105 is guided via endoscopic ultrasound (EUS) to provide access to one or more body lumens or organs associated with the pancreaticobiliary system for the purpose of providing treatment. For example, the system 100 may be configured to provide access to at least the common biliary duct to facilitate subsequent procedures to treat narrowed areas or blockages within the bile duct, including palliative drainage procedures. In accordance with various examples, the system 100 may be used to perform an Endoscopic Ultrasound Guided Biliary Drainage (EUS-BD) procedure. In a particular embodiment, a palliative drainage procedure may be performed in antegrade fashion in conjunction with the system 100. In another embodiment, the palliative drainage procedure may be performed in retrograde fashion, referred to as an Endoscopic Retrograde Cholangiopancreatography (ERCP) "Rendezvous" procedure.

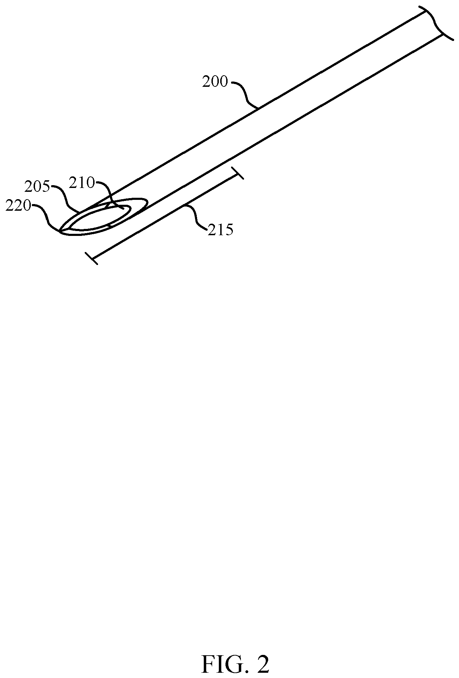

**FIG. 2** illustrates an example of a stylet 200 in accordance with aspects of the present disclosure. The stylet 200 may be an example of aspects of stylet 135 described with reference to FIG. 1. The stylet 200 may be configured to pierce a wall of a body lumen and aspirate fluid from the body lumen or flush contrast into the body lumen. The stylet 200 may generally include a distal end 205 with a lumen 210 extending from a proximal end to the distal end 205 of the stylet 200. The stylet 200 may further include a distal portion 215 with a piercing tip 220.

The stylet 200 may generally be a tubular structure that is sized to fit within the lumen of an access catheter, as described with reference to FIG. 1. In some cases, the cross-section of the distal end 205 of the stylet 200 and the cross-section of the proximal end of the stylet 200 may be circular. The stylet 200 may access the human body through the working channel of the access catheter, for example, as described with reference to FIG. 1.

As will be appreciated, the distal end 205 of the stylet 200 may be formed from any number of bevels to form the piercing tip 220. Furthermore, as will be discussed below, the piercing tip 220 of the stylet 200 may be offset from a circumferential outer surface of the stylet 200. That is, the piercing tip 220 may be biased towards the center line of the stylet 200. As such, the stylet 200 may be configured to avoid catching on the inner lumen of the access catheter as the stylet is advanced through the body lumen of the access catheter.

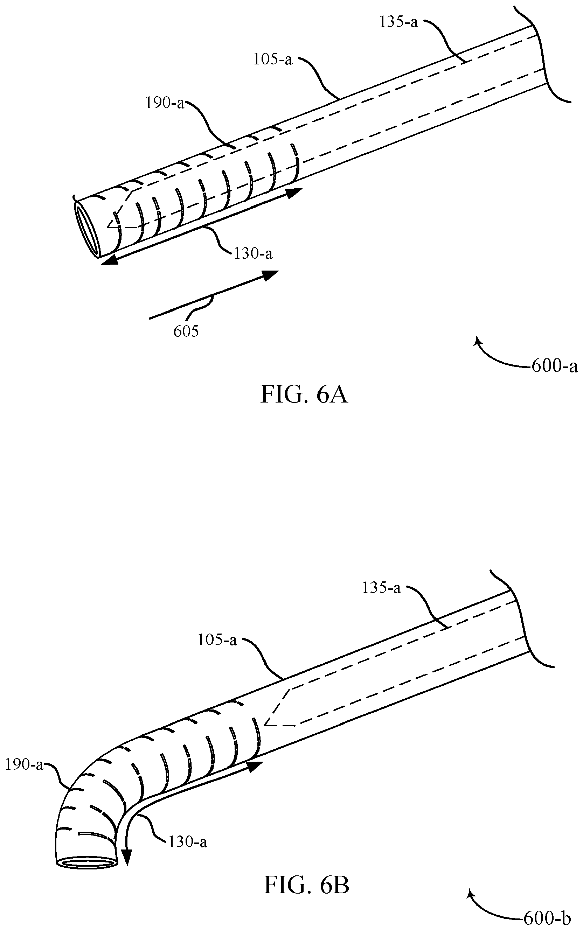

In some examples, the distal end 205 of the stylet 200 may be sharpened to penetrate the tissue without coring. When accessing a body lumen such as the bile duct, for example, the piercing tip 220 of the stylet 200 may be exposed (e.g., protruding from the distal end of the access catheter). In some cases, the stylet 200 and the access catheter may be advanced together through the wall of the tissue, for example, as described with reference to FIG. 6.

As will be discussed below, the stylet 200 may further include a lumen 210 that extends from the proximal end to the distal end 205 of the stylet 200. As such, the stylet 200 may be configured to flush contrast through the lumen 210 without the removal of the stylet 200 from the access catheter. Similarly, the stylet 200 may be configured to aspirate a fluid from the body lumen through the lumen 210 of the stylet 200.

The stylet 200 may be made from any number of materials and combination of materials. In some cases, the circumferential outer surface of the stylet 200 may be coated with a low friction material. For example, the low friction material may be lubricous coating such as parylene. The stylet 200 may comprise a memory shape material to allow the catheter to travel a tortuous path to the tissue site. For example, the memory shape material may be nitinol.

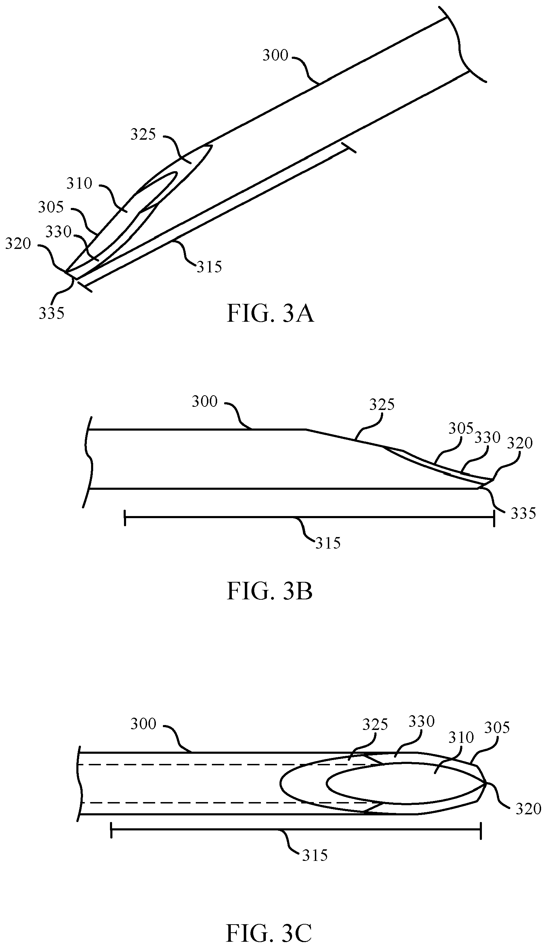

**FIG. 3A** illustrates a perspective view of an stylet 300 in accordance with aspects of the present disclosure. The stylet 300 may be designed to pierce the tissue and aspirate fluid from the body lumen. The stylet 300 may be an example of a stylet 200 described with reference to FIG. 2. In accordance with various examples, the stylet 300 may be used to flush contrast and aspirate fluid without the removal of the stylet 300 from the lumen of the access catheter, as described with reference to FIGs. 1-2. As described above, the stylet 300 may be formed from a number of bevels, such as a first bevel 325 and a second bevel 330. The stylet 300 may further include a distal portion 315 with a piercing tip 320.

In some cases, the stylet 300 may include a piercing tip 320 at the distal end 305 that is offset from the circumferential outer surface of the stylet 300. The piercing tip 320 may be formed by the first bevel 325, the second bevel 330, and a back-cut bevel 335. The stylet 300 may further include a lumen 310 that may extend from the proximal end to the distal end 305 the stylet 300.

**FIG. 3B** illustrates a side view of the stylet 300 of FIG. 3A in accordance with aspects of the present disclosure. The stylet 300 may be manufactured by grinding the material of the stylet 300 to form the piercing tip 320. As described in more detail below, to form the piercing tip 320 of the stylet 300, the distal end 305 may undergo a series of cuts.

In some examples, the distal end 305 of the stylet 300 may include a number of bevels. For example, the first bevel 325 may be formed by a first angle with respect to the longitudinal axis of the stylet 300. As an example, the first angle may be 12 degrees. The second bevel 330 may be formed by a second angle with respect to the longitudinal axis of the stylet 300. For example, the second angle may be 20 degrees. In some cases, the piercing tip 320 may include a back-cut bevel 335. For example, the back-cut bevel 335 may form a back-cut angle with respect to the circumferential outer surface of the stylet 300. The back-cut angle may be 30 degrees. The second bevel 330 and the back-cut bevel 335 may abut at the piercing tip 320. In some cases, the back-cut bevel 335 may prevent the stylet 300 from catching along the access catheter as the stylet 300 advances through the lumen of the access catheter.

**FIG. 3C** illustrates a top view of the stylet 300 in accordance with aspects of the present disclosure. In some cases, the first bevel 325 may include a portion that is processed to reduce the sharpness of the edge created by the first bevel 325 (e.g., a bead blast process). Reducing the sharpness of the edge of the first bevel 325 may help ensure that tissue is not cut and drawn into the lumen 310 of the stylet 300.

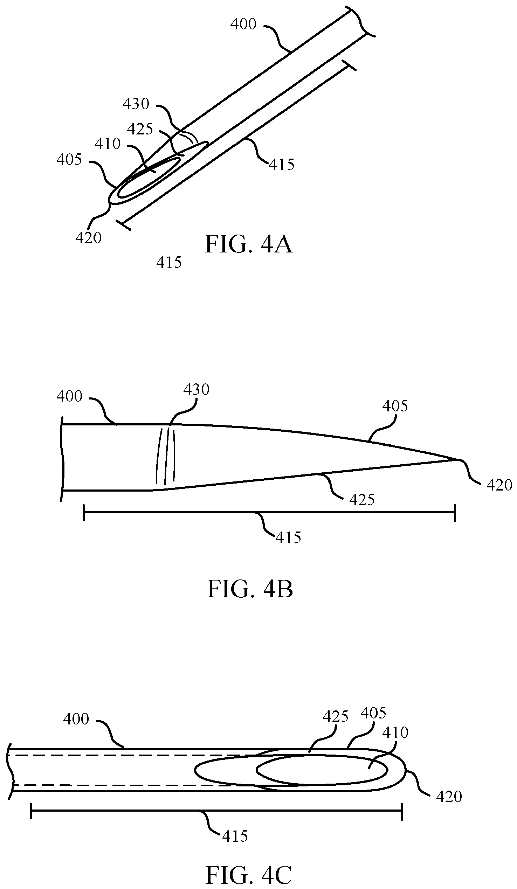

**FIG. 4A** illustrates a perspective view of an stylet 400 in accordance with aspects of the present disclosure. The stylet 400 may be designed to pierce the tissue and aspirate fluid from the body lumen. The stylet 400 may be an example of a stylet 200 described with reference to FIG. 2. In accordance with various examples, the stylet 400 may be used to flush contrast and aspirate fluid without the removal of the stylet 400 from the lumen of the access catheter, as described with reference to FIGs. 1-2. As described above, the stylet 400 may be formed from a single bevel 425.

In some cases, the stylet 400 may include a piercing tip 420 at the distal end 405 that is offset from the circumferential outer surface of the stylet 400. The distal end 405 may be formed by the single bevel 425. In some cases, the distal portion 415 may curve at portion 430 of the stylet 400.

**FIG. 4B** illustrates a side view of the stylet 400 of FIG. 4A in accordance with aspects of the present disclosure. The stylet 400 may be manufactured by grinding the material of the stylet 400 to form the piercing tip 420. As described in more detail below, to form the piercing tip 420 of the stylet 400, the distal portion 415 may be formed by heat shaping (e.g., bending) the material of the stylet 400. In some examples, the distal portion 415 of the stylet 400 may be an example of a Huber tip.

In some examples, the distal end 405 may include a single bevel 425. For example, the single bevel 425 may abut a non-circumferential outer surface of the stylet 400 at the piercing tip 420. In some cases, the distal portion 415 of the stylet 400 may be curved such that a center line of the stylet 400 intersects the piercing tip 420. The stylet 400 may be formed by heat shaping (e.g., bending) the stylet 400 at the distal portion 415 and then grinding the distal end 405 of the stylet 400. As such, the piercing tip 420 may be formed and the lumen of the stylet 400 may be exposed. Alternatively, the stylet 400 may be formed by grinding the distal portion 415 of the stylet 400 to form the piercing tip 420 and then heat shaping (e.g., bending) the distal end 405 of the stylet 400.

**FIG. 4C** illustrates a top view of the stylet 400 in accordance with aspects of the present disclosure. In some cases, the lumen 410 of the stylet 400 may extend from the proximal end to the distal end 405 the stylet 400. Therefore, stylet 400 may be used to flush contrast and aspirate fluid through the lumen 410 without the removal of the stylet 400 from the lumen of the access catheter.

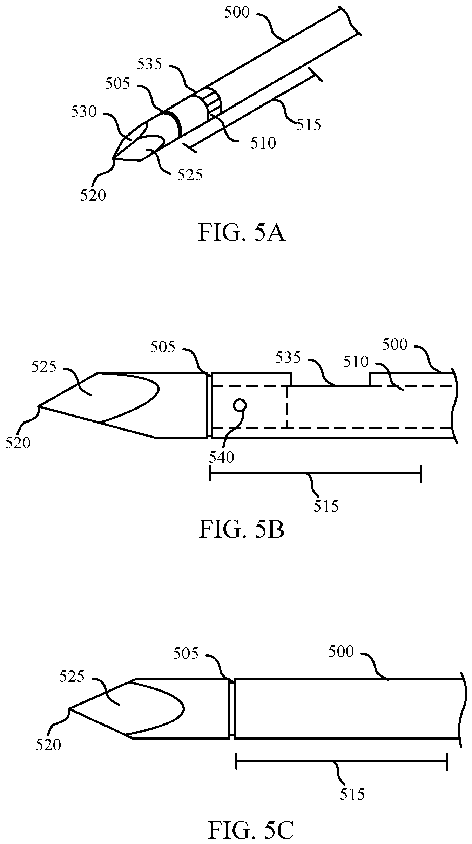

**FIG. 5A** illustrates a perspective view of a stylet 500 in accordance with aspects of the present disclosure. The stylet 500 may be designed to pierce the tissue with the piercing tip 520 and aspirate fluid through one or more apertures 535. The stylet 500 may be an example of a stylet 200 described with reference to FIG. 2. In accordance with various examples, the stylet 500 may be used to flush contrast and aspirate fluid without the removal of the stylet 500 from the lumen of the access catheter, as described with reference to FIGs. 1-2. As described above, the stylet 500 may be formed from a number of bevels, such as a first bevel 525, a second bevel 530, and a third bevel (not shown).

In some cases, the stylet 500 may include a piercing tip 520 at the distal end 505. For example, the piercing tip 520 may intersect a center line of the stylet 500. The stylet 500 may further include one or more apertures 535 disposed along a distal portion 515 of the stylet 500. The apertures 535 may provide fluid access to the lumen 510 since the material that forms the piercing tip 520 blocks the lumen 510 at the distal end 505 of the stylet 500. After the tissue is punctured, the stylet 500 may be advanced forward to expose the apertures 535 and aspirate fluid through the lumen 510 of the stylet 500. That is, the contrast fluid may be flushed through apertures 535 and the fluid from the body lumen may be aspirated through apertures 535.

**FIG. 5B** illustrates a side view of the stylet 500 of FIG. 5A in accordance with aspects of the present disclosure. The stylet 500 may be manufactured by grinding the material of the stylet 500 to form the piercing tip 520 at the distal end 505. In some examples, the material that forms the piercing tip 520 may be a different component that is attached to the stylet 500 at the distal end 505.

The piercing tip 520 may be formed by a number of bevels. For example, the three bevels (first bevel 525, second bevel 530, and third bevel) may form three angles with respect to the longitudinal axis of the stylet 500. The first bevel 525, second bevel (not shown), and third bevel (not shown) may each be at an angle of 15 degrees with respect to the longitudinal axis of the stylet 500. In some cases, the three bevels abut with each other at the piercing tip 520.

The stylet 500 may further include one or more apertures 535 disposed along the distal portion 515 of the stylet 500. The apertures 535 be slits or holes and may extend in any pattern along the distal portion 515 of the stylet 500 and/or around the circumference of the stylet 500. The apertures 535 may be a laser cut to allow fluid to pass into the lumen 510 of the stylet 500. The apertures 535 may extend from the circumferential outer surface of the stylet 500 to an inner surface of the lumen 510 of the stylet 500.

In some cases, the stylet 500 may include one or more apertures 540. Apertures 540 may be equally spaced or unequally spaced around the circumference of the stylet 500 and may be used as welding points to weld or otherwise attach the material that forms the piercing tip 520 to the distal end 505 of the stylet 500.

**FIG. 5C** illustrates a top view of the stylet 500 in accordance with aspects of the present disclosure. In some cases, the distal end 505 may include a larger diameter than the proximal end of the stylet 500 (not shown). For example, the piercing tip 520 may be configured to pierce the tissue and prevent the distal end 505 of the stylet 500 from bouncing back inside the access catheter. The smaller diameter along the body of the stylet 500 may be configured to allow space for fluid to pass between the circumferential outer surface of the stylet 500 and the inner surface of the access catheter.

**FIG. 6A** illustrates a perspective view of an access system 600-a in a linear configuration in accordance with aspects of the present disclosure. The stylet 135-a (shown in phantom lines) may be an example of a stylet described with reference to FIGs. 1-5. In accordance with various examples, the access system 600-a may be used to flush contrast and aspirate fluid without the removal of the stylet 135-a from the lumen of the access catheter 105-a, as described with reference to FIGs. 1-5.

The system 600-a includes a stylet 135-a slidably disposed within the lumen of an access catheter 105-a. In some cases, the stylet 135-a and access catheter 105-a may be advanced together to pierce a wall of the body lumen with the piercing tip of the stylet 135-a. As illustrated in FIGs. 6A and 6B, the distal section 130-a of the access catheter 105-a may be configured to passively transition from a linear configuration (FIG. 6A) to a nonlinear configuration (FIG. 6B) as the stylet 135-a is withdrawn in a proximal direction 605 from the distal section 130-a of the access catheter 105-a. As described with reference to FIG. 1, this passive transition may be achieved by shape setting the distal section 130-a of the access catheter 105-a into a pre-defined nonlinear shape such that in the absence of external constraining forces (i.e., upon removal of the stylet 135-a), the distal section 130-a will transition into the pre-defined nonlinear shape. It may be appreciated that the stiffness of the distal section 130-a relative to the stiffness of the stylet 135-a may be adjusted such that the distal section 130-a conforms to the shape of the stylet 135-a (e.g., linear) while the stylet 135-a is within the distal section 130-a. As shown, the distal section 130-a may include a plurality of apertures 190-a that impart additional flexibility to the distal section 130-a.

In some examples, a clinician may retract the stylet 135-a in a proximal direction 605, thereby causing the distal section 130-a of the access catheter 105-a to curl up into a pre-defined arcuate shape (e.g., as sown in FIG. 6B). In some cases, the distal section 130-a of the stylet 135-a may need to be straightened back out. As such, the stylet 135-a may then be re-advanced distally through the distal section 130-a. As described above, because the sharpened distal end of the stylet 135-a may be offset from an outer circumferential surface of the stylet 135-a, the stylet 135-a may be advanced distally without catching any of the apertures 190-a, and the procedure of accessing a body lumen may resume.

In some cases, the stylet 135-a may be used to flush contrast and aspirate fluid without the removal of the stylet 135-a from access catheter 105-a. This configuration and technique may be advantageous over using the access catheter 105-a alone to aspirate and flush fluid, because in such a case, fluid (e.g., bile) may leak from a body lumen and into the rest of the body via the apertures 190-a.

**FIG. 6B** illustrates a perspective view of an access system 600-b in a nonlinear configuration in accordance with aspects of the present disclosure. The stylet 135-a (shown in phantom lines) may be an example of a stylet described with reference to FIGs. 2-5. In accordance with various examples, the access system 600-a may be used to flush contrast and aspirate fluid without the removal of the stylet 135-a from the lumen of the access catheter 105-a, as described with reference to FIGs. 1-2.

As described herein, access catheter 105-a may include a plurality of apertures 190-a disposed along a length of the distal section 130-a. In general, the apertures 190-a impart flexibility to the distal section 130-a, thereby allowing the distal section 130-a to transition into a nonlinear shape as described with reference to various examples of the present disclosure. In certain examples, the apertures 190-a extend through the entire thickness of the wall of the access catheter 105-a. Alternatively, some or all of the apertures 190-a may only partially penetrate through the wall of the access catheter 105-a.

In accordance with the present disclosure, the size and shape of the apertures 190-a, the spacing of the apertures 190-a, and the overall length of the pattern of apertures 190-a may be optimized to yield certain characteristics that may be advantageous for luminal access and guide wire placement. For example, the apertures 190-a may be uniform in size, shape and spacing, or may be varied as desired to optimize flexibility, radius of curvature, angle of sweep, strength, fatigue resistance, etc.

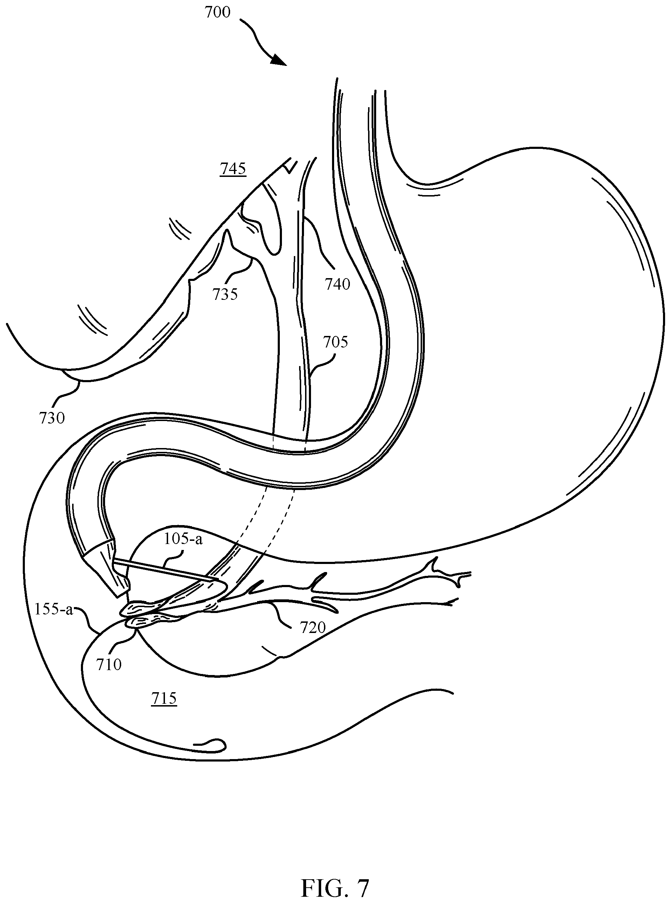

Examples of the present disclosure are now described in the context of a particular Endoscopic Ultrasound Guided Biliary Drainage (EUS-BD) procedure referred to as an Endoscopic Retrograde Cholangiopancreatography (ERCP) "Rendezvous" procedure. With reference to **FIG. 7****,** a system 700 for providing access to a body lumen within the pancreaticobiliary system is illustrated in accordance with various examples. The system 700 may be examples of or include functionality of the systems or components described with reference to any of FIGS. 1-6. The illustrated portions of the pancreaticobiliary system include the common bile duct 705, which drains bile from both the cystic duct 735 (which drains from the gallbladder 730) and the common hepatic duct 740 (which drains from the liver 745) into the duodenum 715, where the bile mixes and reacts with digesting food. As shown, the common bile duct 705 joins with the pancreatic duct 720 at the ampulla of Vater 710 (shown obstructed) before draining through the major duodenal papilla into the duodenum 715.

Under a "Rendezvous" technique, a clinician may advance an endoscope 725 (e.g., an EUS endoscope) into the lumen of a patient's duodenum 715 to a position in which the bile ducts may be visualized (e.g., via endosonography). The clinician may then access the common bile duct 705 by advancing an access catheter 105-a from a working channel of the endoscope 725, through the wall of the duodenum 715 (i.e., trans-duodenally), and then through the wall of the common bile duct 705. As described with reference to FIG. 1, the access catheter 105-a may pierce the wall of the duodenum 715 and the wall of the common bile duct 705 by exposing the distal end of a stylet 135 (not shown for clarity) from the distal end of the access catheter 105-a.

Once at least the distal section of the access catheter 105-a is within the common bile duct 705 (i.e., accessed the bile duct 705), the clinician may then maintain the stylet inside the access catheter 105-a, thereby allowing the clinician to perform a method of aspiration without withdrawing the stylet, as described with reference to FIG. 1.

To verify that the access catheter 105-a is actually within the common bile duct 705, the clinician may use a syringe or vacuum to aspirate fluid from the body lumen and then verify that the aspirated fluid is bile (or any other confirmatory fluid depending on the target lumen or organ). As described with reference to FIGS. 1-6, the clinician may aspirate fluid through the lumen of the stylet. Once proper placement within the common bile duct 705 has been confirmed, the clinician may flush contrast fluid (i.e., fluid visible under fluoroscopy or any other imaging techniques) through the stylet and into the common bile duct 705 to increase the visibility of the biliary lumens and verify proper access catheter insertion of the bile duct 705.

The clinician may then insert a guidewire 155-a into the access catheter 105-a through the proximal end and advance it distally towards the distal end. The clinician may then manipulate the distal end of the access catheter 105-a by rotating the access catheter 105-a or by straightening out or curling up the distal section of the access catheter 105-a. In the case of the described "Rendezvous" procedure, the clinician may rotate the distal section of the access catheter 105-a until the distal end of the access catheter 105-a is facing generally along the antegrade direction of flow of the common bile duct 705 (i.e., in the direction of bile flow from the gallbladder 730 to the duodenum 715). Furthermore, the clinician may adjust the angle of distal portion (i.e., through straightening or curling) of the distal section of the access catheter 105-a by advancing a variable stiffness guidewire 155-a.

After the clinician has manipulated the distal section of the access catheter 105-a to the desired orientation through rotation or straightening, the guidewire 155-a may then be advanced distally from the distal end of the access catheter 105-a and through the bile duct 705 and across the ampulla of Vater 710 into the duodenum 715. In some circumstances, the clinician may advance the access catheter 105-a further into the bile duct 705 over the guidewire 155-a so that the distal end of the access catheter 105-a is closer to the ampulla of Vater 710 or luminal obstruction to be treated to provide additional support for crossing the luminal obstruction. When the guidewire 155-a has passed through the ampulla of Vater 710 and well into the duodenum 715, a "Rendezvous" procedure may be performed, wherein the EUS endoscope 725 and the system 700 are withdrawn from the patient, leaving the guidewire 155-a in place. A side-viewing endoscope (e.g., duodenoscope) may then be passed into the duodenum 715 adjacent the EUS-placed guidewire 155-a. The guidewire 155-a within the duodenum 715 is grasped with a snare or forceps and withdrawn through the duodenoscope. Access to the common bile duct 705 is then performed in reverse fashion over the guidewire 155-a, and a standard ERCP procedure can then be performed (e.g., open blocked ducts, break up or remove gallstones, insert stents, or endoscopic sphincterotomy). It should be noted that EUS-guided biliary access to the common bile duct 705 is not limited to trans-duodenal access, as illustrated in FIG. 7. For example, access to the common bile duct 705 may be achieved trans-gastrically, such that the access catheter 105-a is advanced through the gastric wall and entry into the biliary system could involve the intrahepatic, extrahepatic, or common bile duct 705. In some examples, the system 700 may be used to directly treat (e.g., antegrade stent delivery) the common bile duct 705 directly through the access hole in the bile duct 705 without requiring a "Rendezvous" procedure as described above.

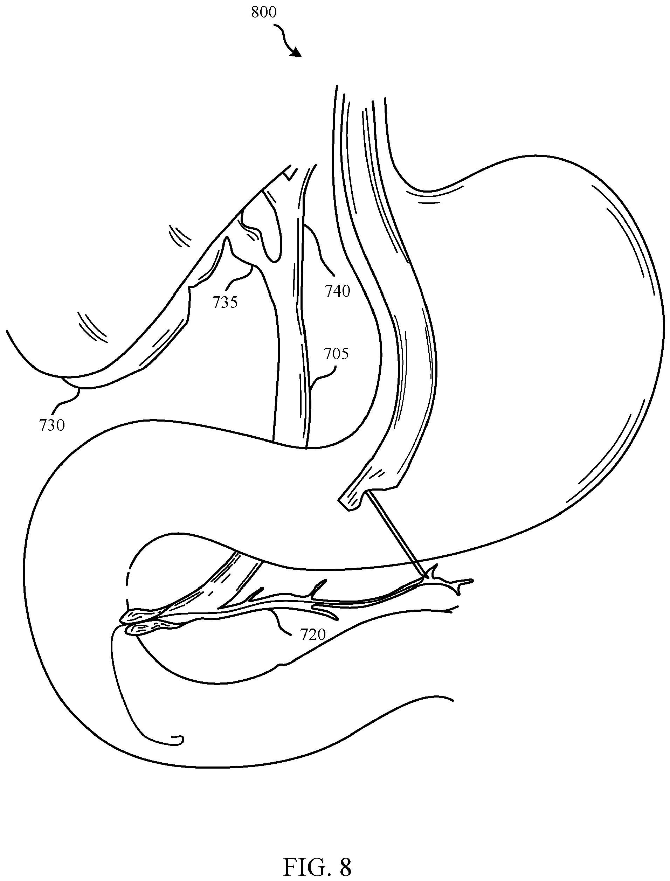

With reference to **FIG. 8****,** the system 800 may be used to directly access the pancreatic duct 720 through the gastric wall. Such a procedure may be advantageous if the treatment site (e.g., obstruction) is located antegrade from where the common bile duct 705 and pancreatic duct 720 join. In addition, the system 800 may be used to directly access the gallbladder 730, the cystic duct 735, the common hepatic duct 740, or any other duct or organ within the pancreaticobiliary system. Moreover, the system 800 may be used to access and treat any other lumen within the body such as those associated with the arterial system, the bronchial system, or the urinary system.

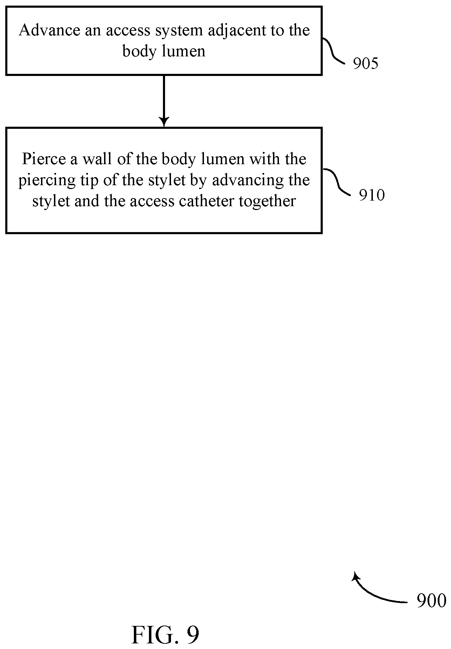

**FIG. 9** illustrates a flowchart of a method 900 of accessing a body lumen in accordance with aspects of the present disclosure. At block 905, the method may include advancing an access system adjacent to the body lumen. The access system may be an example of the access system described with reference to FIGs. 1-6. For example, as described above, the access system may include an access catheter comprising a lumen extending from a proximal end to a distal end of the access catheter and a plurality of apertures disposed along a distal portion of the access catheter. The access catheter may also comprise a stylet configured to fit within the lumen of the access catheter.

At block 910, the method may include piercing a wall of the body lumen with the piercing tip of the stylet by advancing the stylet and the access catheter together. The piercing tip may be an example of any of the piercing tips described with reference to FIGs. 2-5. For example, as described above, the stylet may comprise a lumen extending from a proximal end to a distal end of the stylet and a piercing tip at the distal end of the stylet configured to protrude from the distal end of the access catheter. For example, as described above, the piercing tip may be offset from a circumferential outer surface of the stylet.

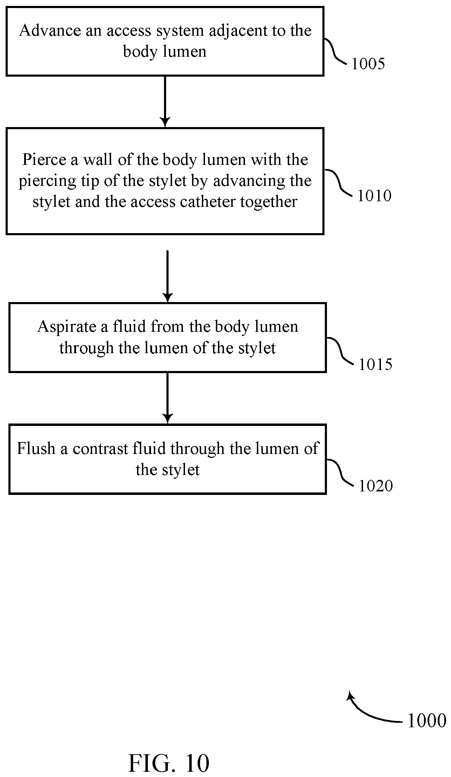

**FIG. 10** illustrates a flowchart of a method 1000 of accessing a body lumen in accordance with aspects of the present disclosure. At block 1005, the method may include advancing an access system adjacent to the body lumen. The access system may be an example of the access system described with reference to FIGs. 1-6. For example, as described above, the access system may include an access catheter comprising a lumen extending from a proximal end to a distal end of the access catheter and a plurality of apertures disposed along a distal portion of the access catheter. The access catheter may also comprise a stylet configured to fit within the lumen of the access catheter.

At block 1010, the method may include piercing a wall of the body lumen with the piercing tip of the stylet by advancing the stylet and the access catheter together. The piercing tip may be an example of any of the piercing tips described with reference to FIGs. 2-5. For example, as described above, the stylet may comprise a lumen extending from a proximal end to a distal end of the stylet and a piercing tip at the distal end of the stylet configured to protrude from the distal end of the access catheter. For example, as described above, the piercing tip may be offset from a circumferential outer surface of the stylet.

At block 1015,the method may include aspirating a fluid from the body lumen through the lumen of the stylet. At block 1020, the method may include flushing a contrast fluid through the lumen of the stylet.

It should be noted that these methods describe possible implementation, and that the operations and the steps may be rearranged or otherwise modified such that other implementations are possible. In some examples, aspects from two or more of the methods may be combined. For example, aspects of each of the methods may include steps or aspects of the other methods, or other steps or techniques described herein.

The description herein is provided to enable a person skilled in the art to make or use the disclosure. Various modifications to the disclosure will be readily apparent to those skilled in the art, and the generic principles defined herein may be applied to other variations without departing from the scope of the disclosure. Thus, the disclosure is not to be limited to the examples and designs described herein but is to be accorded the broadest scope consistent with the principles and novel features disclosed herein.

While several examples of the present disclosure have been described and illustrated herein, those of ordinary skill in the art will readily envision a variety of other means or structures for performing the functions or obtaining the results or one or more of the advantages described herein, and each of such variations or modifications is deemed to be within the scope of the present disclosure. More generally, those skilled in the art will readily appreciate that all parameters, dimensions, materials, and configurations described herein are meant to be exemplary and that the actual parameters, dimensions, materials, or configurations will depend upon the specific application or applications for which the teachings of the present disclosure is/are used.

Those skilled in the art will recognize, or be able to ascertain using no more than routine experimentation, many equivalents to the specific examples of the disclosure described herein. It is, therefore, to be understood that the foregoing examples are presented by way of example only and that, within the scope of the appended claims and equivalents thereto, the disclosure may be practiced otherwise than as specifically described and claimed. The present disclosure is directed to each individual feature, system, article, material, kit, or method described herein. In addition, any combination of two or more such features, systems, articles, materials, kits, or methods, if such features, systems, articles, materials, kits, or methods are not mutually inconsistent, is included within the scope of the present disclosure.

All definitions, as defined and used herein, should be understood to control over dictionary definitions, definitions in documents incorporated by reference, or ordinary meanings of the defined terms.

The indefinite articles "a" and "an," as used herein in the specification and in the claims, unless clearly indicated to the contrary, should be understood to mean "at least one." Also, as used herein, including in the claims, "or" as used in a list of items (for example, a list of items prefaced by a phrase such as "at least one of' or "one or more") indicates an inclusive list such that, for example, a list of at least one of A, B, or C means A or B or C or AB or AC or BC or ABC (i.e., A and B and C).

Reference throughout this specification to "one embodiment" or "an embodiment" means that a particular feature, structure, or characteristic described in connection with the embodiment is included in at least one embodiment. Thus, appearances of the phrases "in one embodiment" or "in an embodiment" in various places throughout this specification are not necessarily all referring to the same embodiment. Furthermore, the particular features, structures, or characteristics may be combined in any suitable manner in one or more examples.

The invention may be described by reference to the following numbered paragraphs:-
1. A method of accessing a body lumen, comprising:
   advancing an access system adjacent to the body lumen, the access system comprising:
      an access catheter comprising:
         a lumen extending from a proximal end to a distal end of the access catheter; and
         a plurality of apertures disposed along a distal portion of the access catheter; and
      a stylet configured to fit within the lumen of the access catheter, the stylet comprising:
         a lumen extending from a proximal end to a distal end of the stylet; and
         a piercing tip at the distal end of the stylet configured to protrude from the distal end of the access catheter; and
   piercing a wall of the body lumen with the piercing tip of the stylet by advancing the stylet and the access catheter together.
2. The method of paragraph 1, wherein the piercing tip is offset from a circumferential outer surface of the stylet.
3. The method of paragraph 1, further comprising:
   aspirating a fluid from the body lumen through the lumen of the stylet.
4. The method of paragraph 1, further comprising:
   flushing a contrast fluid through the lumen of the stylet.
5. The method of paragraph 4, wherein the stylet further comprises a plurality of apertures disposed along a distal portion of the stylet, and wherein the contrast fluid is flushed through the plurality of apertures of the stylet.
6. The method of paragraph 1, wherein the access system further comprises:
   a delivery handle assembly;
   an inner hub housing coupled to a proximal portion of the delivery handle assembly;
   a sheath coupled to a distal portion of the delivery handle assembly and having a lumen in fluid communication with an inner lumen of the delivery handle assembly; and
   an access catheter subassembly removably disposed within the inner lumen of the delivery handle assembly and the lumen of the sheath, the access catheter subassembly comprising:
      a catheter hub configured for insertion into the inner hub housing, wherein the catheter hub is rotatable with respect to the inner hub housing; and
      the access catheter coupled with the catheter hub.
7. The method of paragraph 6, further comprising:
   rotating the catheter hub with respect to the inner hub housing.
8. The method of paragraph 6, wherein the distal portion of the access catheter comprises a pre-defined arcuate shape, the method further comprising:
   straightening the distal portion of the access catheter by advancing the stylet through the distal portion of the access catheter.
9. A system for providing access to a body lumen, comprising:
   an access catheter comprising:
      a lumen extending from a proximal end to a distal end of the access catheter; and
      a plurality of apertures disposed along a distal portion of the access catheter; and
   a stylet configured to fit within the lumen of the access catheter, the stylet comprising:
      a lumen extending from a proximal end to a distal end of the stylet; and
      a piercing tip at the distal end of the stylet.
10. The system of paragraph 9, wherein the piercing tip is offset from a circumferential outer surface of the stylet.
11. The system of paragraph 9, wherein the stylet further comprises:
   a first bevel forming a first angle with respect to a longitudinal axis of the stylet;
   a second bevel forming a second angle with respect to the longitudinal axis of the stylet and abutting the first bevel, wherein the first angle is less than the second angle; and
   a back-cut bevel forming a back-cut angle with respect to a circumferential outer surface of the stylet, wherein the second bevel and the back-cut bevel abut at the piercing tip.
12. The system of paragraph 9, wherein the stylet further comprises:
   a single bevel abutting a non-circumferential outer surface of the stylet at the piercing tip, wherein a distal portion of the stylet is curved such that a center line of the stylet intersects the piercing tip.
13. The system of paragraph 9, wherein the stylet further comprises:
   three bevels forming three equal angles with respect to a longitudinal axis of the stylet and each abutting with each other at the piercing tip.
14. The system of paragraph 13, wherein a center line of the stylet intersects the piercing tip.
15. The system of paragraph 13, wherein the stylet further comprises:
   a plurality of apertures disposed along a distal portion of the stylet, wherein the plurality of apertures extend from a circumferential outer surface of the stylet to an inner surface of the lumen of the stylet.
16. The system of paragraph 9, further comprising:
   a delivery handle assembly;
   an inner hub housing coupled to a proximal portion of the delivery handle assembly;
   a sheath coupled to a distal portion of the delivery handle assembly and having a lumen in fluid communication with an inner lumen of the delivery handle assembly; and
   an access catheter subassembly removably disposed within the inner lumen of the delivery handle assembly and the lumen of the sheath, the access catheter subassembly comprising:
      a catheter hub configured for insertion into the inner hub housing, wherein the catheter hub is rotatable with respect to the inner hub housing; and
      the access catheter coupled with the catheter hub.
17. The system of paragraph 16, further comprising:
   a stylet hub coupled with the proximal end of the stylet and configured to removably couple with the catheter hub, wherein the stylet hub comprises a syringe port in fluid communication with the lumen of the stylet.
18. The system of paragraph 9, wherein a cross-section of the distal end of the stylet and a cross-section of the proximal end of the stylet are circular.
19. The system of paragraph 9, wherein the distal portion of the access catheter comprises a pre-defined arcuate shape, and wherein the distal portion of the access catheter is configured to straighten from the pre-defined arcuate shape as the stylet is advanced through the distal portion of the access catheter.
20. The system of paragraph 9, wherein a circumferential outer surface of the stylet comprises a lubricous coating.

## Claims

1. A system for providing access to a body lumen, comprising:
an access catheter comprising:
a lumen extending from a proximal end to a distal end of the access catheter; and
a plurality of apertures disposed along a distal portion of the access catheter; and
a stylet configured to fit within the lumen of the access catheter, the stylet comprising:
a lumen extending from a proximal end to a distal end of the stylet; and
a piercing tip at the distal end of the stylet.

2. The system of claim 1, wherein the piercing tip is offset from a circumferential outer surface of the stylet.

3. The system of claim 1 or 2, wherein the stylet further comprises:
a first bevel forming a first angle with respect to a longitudinal axis of the stylet;
a second bevel forming a second angle with respect to the longitudinal axis of the stylet and abutting the first bevel, wherein the first angle is less than the second angle; and
a back-cut bevel forming a back-cut angle with respect to a circumferential outer surface of the stylet, wherein the second bevel and the back-cut bevel abut at the piercing tip.

4. The system of claim 1 or 2, wherein the stylet further comprises:
a single bevel abutting a non-circumferential outer surface of the stylet at the piercing tip, wherein a distal portion of the stylet is curved such that a center line of the stylet intersects the piercing tip.

5. The system of claim 1, wherein the stylet further comprises:
three bevels forming three equal angles with respect to a longitudinal axis of the stylet and each abutting with each other at the piercing tip.

6. The system of claim 5, wherein a center line of the stylet intersects the piercing tip.

7. The system of claim 5 or 6, wherein the stylet further comprises:
a plurality of apertures disposed along a distal portion of the stylet, wherein the plurality of apertures extend from a circumferential outer surface of the stylet to an inner surface of the lumen of the stylet.

8. The system of any preceding claim, further comprising:
a delivery handle assembly;
an inner hub housing coupled to a proximal portion of the delivery handle assembly;
a sheath coupled to a distal portion of the delivery handle assembly and having a lumen in fluid communication with an inner lumen of the delivery handle assembly; and
an access catheter subassembly removably disposed within the inner lumen of the delivery handle assembly and the lumen of the sheath, the access catheter subassembly comprising:
a catheter hub configured for insertion into the inner hub housing, wherein the catheter hub is rotatable with respect to the inner hub housing; and
the access catheter coupled with the catheter hub.

9. The system of claim 8, further comprising:
a stylet hub coupled with the proximal end of the stylet and configured to removably couple with the catheter hub, wherein the stylet hub comprises a syringe port in fluid communication with the lumen of the stylet.

10. The system of any preceding claim, wherein a cross-section of the distal end of the stylet and a cross-section of the proximal end of the stylet are circular.

11. The system of any preceding claim, wherein the distal portion of the access catheter comprises a pre-defined arcuate shape, and wherein the distal portion of the access catheter is configured to straighten from the pre-defined arcuate shape as the stylet is advanced through the distal portion of the access catheter.

12. The system of any preceding claim, wherein a circumferential outer surface of the stylet comprises a lubricous coating.

13. The system of any preceding claim further comprising means for aspirating a fluid from the body lumen through the lumen of the stylet.

14. The system of any preceding claim, further comprising means for flushing a contrast fluid through the lumen of the stylet.
